# EUROPÄISCHE PATENTANMELDUNG

(11) **EP 0 588 759 A1**
(43) Veröffentlichungstag der Anmeldung: **23.03.1994**
(21) Anmeldenummer: 93810572.3
(22) Anmeldetag: 11.08.1993
(51) Int. Cl.: C07D 339/06, C07D 339/08, C07D 495/04, C08K 5/45, C08K 5/48

(54) **Dithiopentacenderivate, deren Herstellung und deren Verwendung als Elektronenakzeptoren in Charge-Transfer Komplexen**

(30) Priorität: 20.08.1992 CH 2591/92
(71) Anmelder: CIBA-GEIGY AG, CH-4002 Basel (CH)
(72) Erfinder: Chetcuti, Peter Dr., CH-4052 Basel (CH)

(57) **Zusammenfassung**

Die Erfindung betrifft neue Dithiopentacenderivate; ein Verfahren zu deren Herstellung; Charge-Transfer Komplexe aus solchen Dithiopentacenderivaten als Elektronenakzeptoren und unsubstituiertem oder substituiertem Ferrocen, chalkogenierten Fulvalenen oder Stickstoff-aromatischen Verbindungen als Elektronendonatoren; ein Verfahren zu deren Herstellung; Zusammensetzungen aus einem Kunststoff und einem solchen Charge-Transfer Komplex; und die Verwendung der Charge-Transfer Komplexe als elektrische Leiter, zum Beispiel zur Herstellung von elektrisch leitenden Filmen, Folien oder Beschichtungen.

## Beschreibung

Die Erfindung betrifft neue Dithiopentacenderivate; ein Verfahren zu deren Herstellung; Charge-Transfer Komplexe (nachfolgend als CT-Komplexe abgekürzt) aus solchen Dithiopentacenderivaten als Elektronenakzeptoren und unsubstituiertem oder substituiertem Ferrocen, chalkogenierten Fulvalenen oder Stickstoff-aromatischen Verbindungen als Elektronendonatoren; ein Verfahren zu deren Herstellung; Zusammensetzungen aus einem Kunststoff und einem solchen CT-Komplex; und die Verwendung der CT-Komplexe als elektrische Leiter, zum Beispiel zur Herstellung von elektrisch leitenden Filmen, Folien oder Beschichtungen.

Die Verbindung Dinaphtho[2,3-b;2',3'-e][1,4]dithiin-5,7,12,14-tetraon (als DNDT abgekürzt) wird von E.S. Martinez et al. in J. Chem. Research (S), S.246-247 (1991) beschrieben. DNDT an sich weist bei Raumtemperatur eine sehr niedrige elektrische Leitfähigkeit von 10⁻¹¹ S/cm als Pressling auf. Ferner wird ein Herstellungsverfahren von K. Brass und L. Köhler in Ber. Deutsch. Chem. Ges., 55, S.2543-2568 (1992) beschrieben. Dinaphtho[2,3-b;2',3'-e][1,4]dithiin-one bzw. -cyanoimine sind nicht beschrieben.

In Synthetic Metals, 41-43, Seiten 2365 bis 2375 (1991) wird ein pulverförmiger CT-Komplex aus Tetracyanoiminpentacen und Tetrathiofulvalen als Elektronendonator, sowie Einzelkristalle aus Tetracyanoiminpentacen und Alkalimetallkationen und Tetraalkylammonium beschrieben. Pulverförmige Materialien können die elektrischen Leitfähigkeiten von Kunststoffen jedoch nur ungenügend verbessern, da die Teilchen nach der Einarbeitung vom Kunststoff umhüllt und damit isoliert werden.

Ferner wird 5,7,12,14-Tetracyanoiminpentacen von L. Miller et al. in Chem. Mater. 2, Seiten 339-40 (1990) als Elektronenakzeptor zur Herstellung von Radikalkationensalzen mit Alkalimetallen wie zum Beispiel Natrium und Kalium beschrieben.

Im US Patent 5,009,812 werden antistatisch ausgerüstete und elektrisch leitfähige Polymere beschrieben, die zum Beispiel CT-Komplexe aus Tetrathio-, Tetraseleno- oder Tetratellurotetracenen als Elektronendonatoren und Halogenen oder Sauerstoff als Elektronenakzeptoren enthalten. Die CT-Komplexe bilden in diesen Materialien Nadelnetzwerke in der Polymermatrix.

CT-Komplexe aus Tetracyanochinodimethan (TCNQ) als Elektronenakzeptor und N-enthaltenden aromatischen Verbindungen als Donatoren sind, z.B. von C. D. Jaeger und A. J. Bard in J. Am. Chem. Soc., Vol. 102, Nr. 17, Seiten 5435-5442 (1980), und von L. Russell Melby in Can. J. Chem., Vol. 43, Seiten 1448-1453 (1965) beschrieben. Diese CT-Komplexe kristallisieren nicht immer nadelförmig, und sind auf Grund ihrer Kristallgestalt nicht zur Herstellung von elektrisch leitenden Folien mit einem Netzwerk aus Kristallnadeln geeignet.

Es wurde nun gefunden, dass 6,13-Dithiopentacenderivate und unsubstituiertes Ferrocen oder bestimmte substituierte Ferrocene (nachfolgend als Ferrocenderivate abgekürzt), Fulvalenderivate oder N-aromatische Verbindungen überraschend CT-Komplexe bilden, die unerwartet selbst in Gegenwart von Bindemitteln nadelförmig kristallisieren, eine hohe elektrische Leitfähigkeit aufweisen und praktisch keine korrosive Wirkung auf metallische Teile von Verarbeitungsmaschinen zeigen. Die Ausgangsverbindungen sind auch in weniger polaren organischen Lösungsmitteln löslich, so dass zur Herstellung der CT-Komplexe keine sehr hohen Temperaturen angewendet werden müssen. Die CT-Komplexe weisen eine unerwartet hohe Stabilität gegenüber Feuchtigkeit und Wärme auf. Ferner bilden die CT-Komplexe überraschend feine und stabile Kristallnadeln aus, wodurch Filme oder Folien mit sehr feinmaschigen Nadelnetzwerken und hoher elektrischer Leitfähigkeit erhalten werden.

Ein Gegenstand der Erfindung sind Verbindungen der Formel I 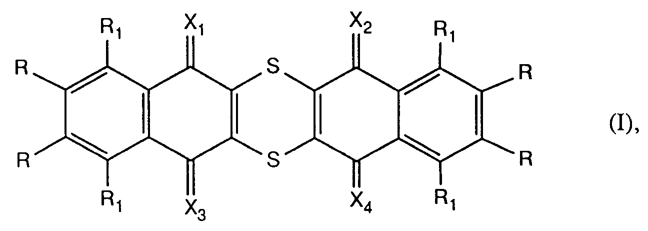
worin die R gleich oder verschieden sind und für H oder C₁-C₄-Alkyl stehen, oder die benachbarten R zusammen -(CH₂)₃- oder -(CH₂)₄- darstellen; die R₁ gleich oder verschieden sind und H oder C₁-C₄-Alkyl bedeuten; und X₁ =N-CN darstellt und X₂, X₃ und X₄ =O oder =N-CN bedeuten.

Bei R und R₁ in der Bedeutung von Alkyl kann es sich um Methyl, Ethyl, n- oder i-Propyl oder n-, i- oder t-Butyl handeln. Bevorzugte Alkylreste sind Methyl und Ethyl. In einer bevorzugten Ausführungsform stellen die R C₁-C₄-Alkyl und die R₁ H dar, oder stellen die R₁ C₁-C₄-Alkyl und die R H dar. Bevorzugt stellen R und R₁ H, Methyl oder Ethyl dar. In einer besonders bevorzugten Ausführungsform bedeuten R und R₁ H.

In einer anderen bevorzugten Ausführungsform bedeuten X₁ und X₄ =N-CN und X₂ und X₃ =O oder =N-CN, oder X₂ und X₃ =N-CN und X₁ und X₄ =O oder =N-CN. Besonders bevorzugt stellen X₁, X₂, X₃ und X₄ =N-CN dar.

Bevorzugte Verbindungen der Formel I sind Dinaphtho[2,3-b;2',3'-e][1,4]dithiin-5,7,12,14-tetracyanoimin, 2,3,9,10-Tetramethyl-dinaphtho[2,3-b;2',3'-e][1,4]dithiin-5,7,12,14-tetracyanoimin und 2,9-Dimethyl-dinaphtho[2,3-b;2',3'-e][1,4]dithiin-5,7,12,14-tetracyanoimin.

Ein anderer Gegenstand der Erfindung ist ein Verfahren zur Herstellung von Verbindungen der Formel I, dadurch gekennzeichnet, dass man ein Tetraon der Formel Ia 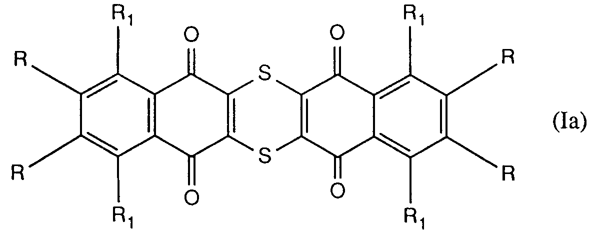
mit äquimolaren Mengen TiCl₄ und [(CH₃)₃SiN]₂C in einer inerten Atmosphare und in einem inerten Lösungsmittel für 10 bis 30 Stunden erhitzt. In dieser Arbeitsweise wird DNDT ins Dinaphtho[2,3-b;2',3,-e][1,4]dithiin-5,7,12,14-cyanoimin (6,13-Dithio-pentacentetracyanoimin) umgesetzt.

Das Lösungsmittel ist vorzugsweise ein halogenierter Kohlenwasserstoff, zum Beispiel Dichlormethan oder Dichlorethan.

Argon wird vorzugsweise als inerte Atmosphäre verwendet.

Die Reaktionstemperatur kann zum Beispiel zwischen 0 und 100 °C liegen.

Es wird mindestens 1 Äquivalent TiCl₄ und 1 Äquivalent [(CH₃)₃SiN]₂C pro "-on" Gruppe verwendet. Vorzugsweise werden Überschüsse verwendet, zum Beispiel ein bis zu fünffacher und bevorzugt ein bis zu dreifacher Überschuss.

Die Herstellung von 6,13-Dithio-5,7,12,14-tetraon (Dinaphtho[2,3-b;2',3',-e][1,4]dithiin-5,7,12,14-tetraon) ist bekannt und kann nach dem von K.Brass und L. Köhler in Ber. Deutsch. Chem. Ges. 55, S. 2543 (1992) beschriebenen Verfahren erfolgen. Die entsprechenden substituierten Tetraone können in analoger Weise hergestellt werden.

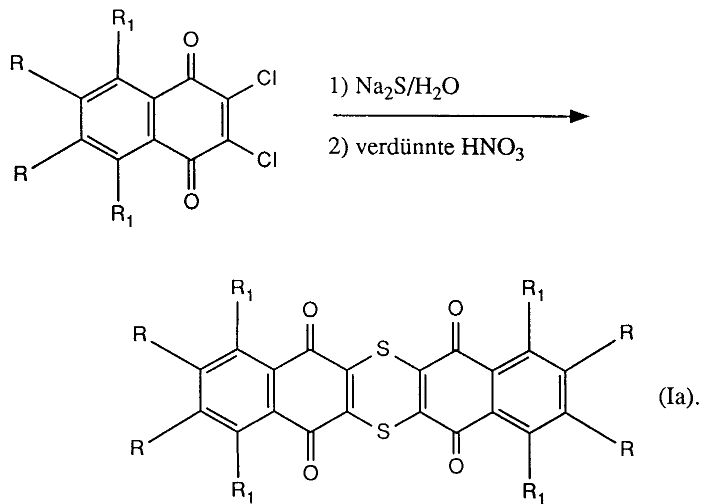

Verbindungen der Formel I eignen sich hervorragend zur Bildung von elektrisch leitenden Charge-Transfer Komplexen.

Ein anderer Gegenstand der Erfindung sind Charge-Transfer Komplexe der Formel II

[(A)p]^{q⊖}B^{q⊕} (II),

worin
a) A das Radikalanion einer Verbindung der Formel I oder einer Mischung von Verbindungen der Formel I ist, und
b) p und q gleich 1 sind und
   B das einwertige Radikalkation einer Verbindung der Formel IIIa oder IIIb bedeutet 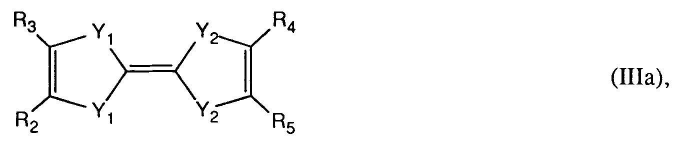 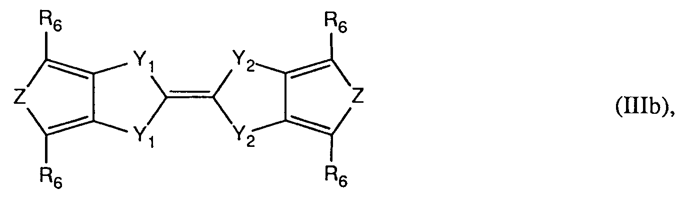 worin R₂, R₃, R₄ und R₅ unabhängig voneinander H, lineares oder verzweigtes C₁-C₁₈-Alkyl-(Z₁)ₙ-, unsubstituiertes oder mit C₁-C₄-Alkyl, C₁-C₄-Alkoxy, C₁-C₄-Alkylthio substituiertes Phenyl-(Z₁)ₙ- oder Benzyl-(Z₁)ₙ- bedeuten, oder R₂ und R₃ sowie R₄ und R₅ je zusammen unabhängig voneinander unsubstituiertes oder mit C₁-C₄-Alkyl, C₁-C₄-Alkoxy, C₁-C₄-Alkylthio substituiertes Trimethylen, Tetramethylen, -Z₂-(CH₂)-Z₂-, -Z₂-(CH₂)₂-Z₂-, -Z₁-CH=CH-Z₁- oder -CH=CH-CH=CH- darstellen, n für 0 oder 1 steht, Y₁ und Y₂ unabhängig voneinander -S- oder -Se- sind, Z₁ für -S- oder -Se- steht, Z₂ -O-, -S- oder -Se- darstellt, Z -S-, -Se- oder -NR₇- bedeutet und R₇ H, C₁-C₆-Alkyl, Phenyl oder Benzyl ist, und R₆ für H, C₁-C₄-Alkyl, Phenyl oder Benzyl steht; oder
   das einwertige Radikalkation einer N-aromatischen Verbindung mit insgesamt 1 bis 5 unsubstituierten oder mit Halogen, C₁-C₄-Alkyl oder C₁-C₄-Alkoxy substituierten aromatischen Ringen ist, wobei mindestens ein Ring mindestens eine Gruppe -NR₈- oder [=N⁺R₈-] I⁻ enthält, worin R₈ C₁-C₄-Alkyl oder Benzyl ist;
   oder
c) p und q gleich 2 sind und
   B das zweiwertige Radikalkation einer wie oben erwähnten N-aromatischen Verbindung ist; oder
d) p gleich 2 und q gleich 1 ist und
   B unsubstituiertes Ferrocen oder Fe(Indenyl)₂, oder ein mit C₁-C₆-Alkyl, C₁-C₆-Alkoxy, C₁-C₆-Hydroxyalkyl, -Amino-C₁-C₆-alkyl, Primär- oder Sekundäramino-C₁-C₆-alkyl mit 1 bis 12 C-Atomen in der Primäraminogruppe und 2 bis 12 C-Atomen in der Sekundäraminogruppe, NH₂, Primäramino mit 1 bis 12 C-Atomen, oder Sekundäramino mit 2 bis 12 C-Atomen substituiertes Ferrocen oder Fe(Indenyl)₂ ist.

Für R, R₁ und X₁ bis X₄ gelten die zuvor angegebenen Bevorzugungen.

In einer bevorzugten Ausführungsform handelt es sich bei Y₁ und Y₂ in den Verbindungen der Formeln IIIa und IIIb entweder um -S- oder um -Se-, und besonders bevorzugt um -S-.

In den Verbindungen der Formel IIIa stellen R₂ und R₃ sowie R₄ und R₅ bevorzugt gleiche Reste dar und besonders bevorzugt handelt es sich bei R₂ bis R₅ um gleiche Reste.

Bei R₂ bis R₅ in der Bedeutung von Alkyl-(Z₁)ₙ- handelt es sich bevorzugt um C₁-C₁₂-Alkyl-(Z1)ₙ-, besonders bevorzugt um C₁-C₈-Alkyl-(Z₁)-ₙ und insbesondere bevorzugt um C₁-C₄-Alkyl-(Z₁)-ₙ. Bevorzugt ist das Alkyl linear. Beispiele für Alkyl sind Methyl, Ethyl, n- und i-Propyl, n-, i- und t-Butyl, Pentyl, Hexyl, Heptyl, Octyl, Nonyl, Decyl, Undecyl, Dodecyl, Tetradecyl, Hexadecyl und Octadecyl. Besonders bevorzugt sind Methyl, Ethyl, n- und i-Propyl, n-, i- und t-Butyl.

In einer bevorzugten Ausgestaltung handelt es sich bei dem Rest Alkyl-(Z₁)ₙ- um Methyl, Ethyl, n- und i-Propyl, n-, i- und t-Butyl, Methylthio, Methylseleno, Ethylthio und Ethylseleno.

Bei den Substituenten C₁-C₄-Alkyl, C₁-C₄-Alkoxy und C₁-C₄-Alkylthio kann es sich zum Beispiel um Methyl, Ethyl, n- und i-Propyl, n-, i- und t-Butyl sowie um entsprechende Alkoxy und Alkylthioreste handeln. Bevorzugte Substituenten sind Methyl, Ethyl, n- und i-Propyl, n- und i-Butyl, Methoxy, Ethoxy, Methylthio und Ethylthio.

Einige Beispiele für die Reste Phenyl-(Z₁)ₙ- oder Benzyl-(Z₁)ₙ- sind Phenyl, Benzyl, Phenylthio, Phenylseleno, Benzylthio, Benzylseleno, Methylphenyl, Methylbenzyl, Ethylphenyl, n- oder i-Propylphenyl, n-, i- oder t-Butylphenyl, Dimethylphenyl, Dimethylbenzyl, Methoxyphenyl, Methylthiophenyl, Methylthiobenzyl, Methylphenylthio und Methylphenylseleno.

In Formel IIIb stellt R₆ bevorzugt H oder C₁-C₄-Alkyl dar, und besonders bevorzugt ist R₆ H, Methyl oder Ethyl.

Z in Formel IIIb steht bevorzugt für -S- oder -NR₇- und besonders bevorzugt für -NR₇-. R₇ bedeutet bevorzugt H oder C₁-C₄-Alkyl, und besonders bevorzugt ist R₇ H, Methyl oder Ethyl.

Z₁ bedeutet bevorzugt -S- und Z₂ stellt bevorzugt -O- oder -S- dar.

Eine bevorzugte Untergruppe der Verbindungen der Formel II sind solche, worin in Formel I R H, Methyl oder Ethyl und besonders bevorzugt H bedeutet, R₁ H oder Methyl und bevorzugt H darstellt und X₁ bis X₄ für =N-CN stehen, und in den Formeln IIIa und IIIb R₂ und R₃ sowie R₄ und R₅ oder R₂ bis R₅ gleich sind und H, lineares oder verzweigtes C₁-C₈-Alkyl-(Z₁)ₙ-, unsubstituiertes oder mit C₁-C₄-Alkyl substituiertes Phenyl-(Z₁)ₙ- oder Benzyl-(Z₁)ₙ- bedeuten, oder R₂ und R₃ sowie R₄ und R₅ je zusammen unabhängig voneinander unsubstituiertes oder mit C₁-C₄-Alkyl substituiertes Trimethylen, Tetramethylen, -Z₂-(CH₂)-Z₂-, -Z₂-(CH₂)₂-Z₂-, -Z₁-CH=CH-Z₁- oder -CH=CH-CH=CH- darstellen, n für 0 oder 1 steht, Y₁ und Y₂ für -S- stehen, Z₁ für -S- steht, Z₂-O- oder -S- darstellt, Z -S- oder NR₇ bedeutet und R₇ H oder C₁-C₄Alkyl ist, und R₆ für H oder C₁-C₄-Alkyl steht.

Eine besonders bevorzugte Untergruppe der Verbindungen der Formel II sind solche, worin in Formel I R und R₁ H bedeuten, X₁ bis X₄ für =N-CN stehen, und in den Formeln IIIa und IIIb R₂ bis R₅ gleich sind und H, oder lineares oder verzweigtes C₁-C₄-Alkyl-(Z₁)ₙ- darstellen, oder R₂ und R₃ sowie R₄ und R₅ je zusammen Trimethylen, Tetramethylen, -Z₂-(CH₂)-Z₂-, -Z₂-(CH₂)₂-Z₂-, -Z₁-CH=CH-Z₁- oder -CH=CH-CH=CH- bedeuten, n für 0 oder 1 steht, Y₁ und Y₂ für -S- stehen, Z₁ für -S- steht, Z₂ -O- oder -S- darstellt, Z -S- oder NR₇ bedeutet und R₇ H oder C₁-C₄-Alkyl ist, und R₆ für H oder C₁-C₄-Alkyl steht.

Einige Beispiele für CT-Komplexe der Formel Il sind solche, worin A in Formel Il 6,13-Dithio-5,7,12,14-tetracyanoiminpentacen ist, und B Tetrathiofulvalen, Tetramethyltetrathiofulvalen, Tetraethyltetrathiofulvalen, Dimethyl-diethyl-tetrathiofulvalen, Tetra-n-propyl-tetrathiofulvalen, Tetra-n-butyl-tetrathiofulvalen, Tetra(methylthio)tetrathiofulvalen, Tetra(ethylthio)tetrathiofulvalen, Tetra(n-propylthio)tetrathiofulvalen, Tetra(n-butylthio)tetrathiofulvalen, Dimethyl-dimethylthiotetrathiofulvalen, Diethyl-dimethylthiotetrathiofulvalen, Diethylthio-dimethylthiotetrathiofulvalen und Tetraselenofulvalen bedeutet.

Wenn B das Radikalkation einer N-aromatischen Verbindung bedeutet, kann es sich bei den aromatischen Ringen um Kohlenwasserstoff-Ringe oder N-heterocyclische Ringe mit einem oder zwei N-Atomen handeln.

Als eine N-aromatische Verbindung enthält B bevorzugt insgesamt 1 bis 3 Ringe und mindestens einen N-aromatischen Ring. In einer bevorzugten Ausführungsform enthält B 1 bis 3 Ringe und einen heteroaromatischen Ring oder B stellt einen bis-N-heteroaromatischen Ring dar. Die Ringe sind bevorzugt 6-gliedrig. Besonders bevorzugte N-Aromaten sind Pyridin, Pyrimidin, Pyrazin und Phenazin.

In einer besonders bevorzugten Ausführungsform entspricht B als N-aromatische Verbindung in Formel II Kationen der Formeln IVa bis IVf: 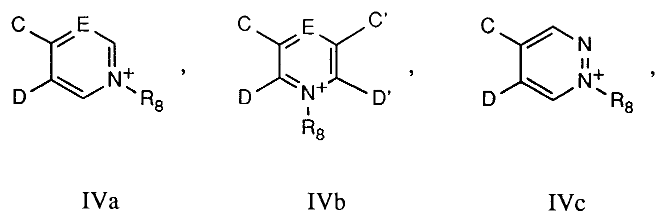 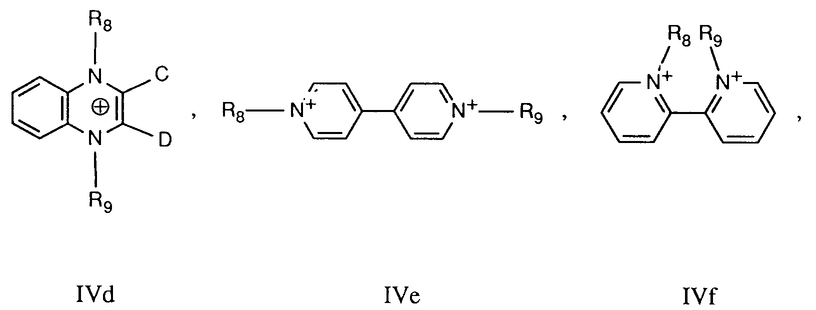
worin C, C', D und D' für H stehen oder C und D und/oder C' und D' die Gruppe -CH=CH-CH=CH- bedeuten, E N oder CH bedeutet, und R₉ unabhängig die gleiche Bedeutung wie R₈ hat, wobei R₈ für C₁-C₄-Alkyl oder Benzyl steht.

Bei R₈ handelt es sich bevorzugt um Ethyl oder Methyl.

Bei B als N-aromatische Verbindung in Formel II kann es sich zum Beispiel um Kationen von N-Methyl- und N-Ethyl-pyridinium; N-Methyl- und N-Ethyl-pyrazinium; N-Methyl- und N-Ethyl-chinolinium; N-Methyl- und N-Ethyl-phthalazinium; N-Methyl und N-Ethyl-isochinolinium; N-Methyl- und N-Ethyl-benzopyrazinium; 4,4'-Dimethyl-, 4,4'-Diethyl- und 4-Methyl,4'-ethyl-bipyridinium; N-Methyl- und N-Ethyl-acridinium; N-Methyl- und N-Ethyl-phenazinium; 2,2'-Dimethyl-, 2,2'-Diethyl- und 2-Methyl,2'-ethyl-bipyridinium; N-Methyl- oder N-Ethylpyridazinium; 5,10-Dimethyl-, 5,10-Diethyl- oder 5-Methyl,10-ethyl-5,10-dihydrophenazinium handeln.

Die Verbindung der Formel I ist bevorzugt 6,13-Dithio-5,7,12,14-tetracyanoiminpentacen (Dinaphtho[2,3-b;2',3'-e][1,4]dithiin-5,7,12,14-tetracyanoimin), das in reiner Form vorliegt, oder bis zu 10 Gew-%, bezogen auf die Gesamtmischung, Verbindungen der Formel I enthält, in denen ein oder zwei Cyanoimingruppen durch Sauerstoff ersetzt sind. Besonders bevorzugte CT-Komplexe der Formel II sind solche aus 6,13-Dithio-5,7,12,14-tetracyanoiminpentacen und N-Methylpyrazinium, N-Ethylpyrazinium oder 5-Methyl,10-ethyl-5,10-dihydrophenazinium als B.
Bei B als Ferrocenderivat in Formel II kann es sich zum Beispiel um Fe[R₁₀]₂ handeln, worin R₁₀ für Cyclopentadienyl oder Indenyl steht, die 1 bis 5 beziehungsweise 1 bis 7 Substituenten aus der Gruppe C₁-C₆-Alkyl, C₁-C₆-Alkoxy, C₁-C₆-Hydroxyalkyl, -Amino-C₁-C₆-alkyl, Primär- oder Sekundäramino-C₁-C₆-alkyl mit 1 bis 12 C-Atomen in der Primäraminogruppe und 2 bis 12 C-Atomen in der Sekundäraminogruppe, NH₂, Primäramino mit 1 bis 12 C-Atomen, oder Sekundäramino mit 2 bis 12 C-Atomen enthalten.

Das Alkyl kann linear oder verzweigt sein und es enthält bevorzugt 1 bis 4 C-Atome. Beispiele sind Methyl, Ethyl, n- oder i-Propyl, n-, i- oder t-Butyl, Pentyl und Hexyl. Bevorzugt sind Ethyl und besonders Methyl.

Das Alkoxy kann linear oder verzweigt sein und es enthält bevorzugt 1 bis 4 C-Atome. Beispiele sind Methoxy, Ethoxy, n- oder i-Propoxy, n-, i- oder t-Butoxy, Pentoxy und Hexoxy. Bevorzugt sind Ethoxy und besonders Methoxy.

Das Hydroxyalkyl kann linear oder verzweigt sein und es enthält bevorzugt 1 bis 4 C-Atome. Beispiele sind Hydroxymethyl, Hydroxyethyl, n- oder i-Hydroxypropyl, n-, i- oder t-Hydroxybutyl, Hydroxypentyl und Hydroxyhexyl. Bevorzugt sind Hydroxyethyl und besonders Hydroxymethyl.

Das Aminoalkyl kann linear oder verzweigt sein und es enthält bevorzugt 1 bis 4 C-Atome. Beispiele sind Aminomethyl, Aminoethyl, n- oder i-Aminopropyl, n-, i- oder t-Aminobutyl, Aminopentyl und Aminohexyl. Bevorzugt sind Aminoethyl und besonders Aminomethyl.

Das Primär- und Sekundäraminoalkyl kann linear oder verzweigt sein und es enthält bevorzugt 1 bis 4 C-Atome. Die Primäraminogruppe enthält bevorzugt eine C₁-C₆-Alkylgruppe, besonders bevorzugt eine C₁-C₄-Alkylgruppe und die Sekundäraminogruppe enthält bevorzugt zwei C₁-C₆-Alkylgruppen, besonders bevorzugt eine C₁-C₄-Alkylgruppen; besonders bevorzugte Alkylgruppen sind Methyl und Ethyl. Beispiele sind Methylaminomethyl, Dimethylamino-methyl, Diethylamino-methyl, Methylamino-ethyl, Dimethylamino-ethyl, oder Diethylamino-propyl, Dimethylamino-butyl, Diethylamino-pentyl und Dimethylamino-hexyl. Bevorzugt sind Methylamino-ethyl, Dimethylamino-ethyl, Ethylamino-ethyl, Diethylamino-ethyl, Methylamino-methyl, Dimethylamino-methyl, Ethylamino-methyl und Diethylamino-methyl.

Das Primäramino enthält bevorzugt 1 bis 6 C-Atome und das Sekundäramino enthält bevorzugt 2 bis 6 C-Atome. Vorzugsweise enthält das Primär- und das Sekundäramino C₁-C₄-Alkyl, zum Beispiel Methyl, Ethyl, n- oder i-Propyl oder n-, i- oder t-Butyl. Einige Beispiele sind Methylamino, Dimethylamino, Ethylamino, Diethylamino, n- und i-Propylamino, Di-n- und Di-i-propylamino, n-, i- und t-Butylamino, Di-n- und Di-i-butylamino.

In einer Ausführungsform steht R₁₀ für Cyclopentadienyl oder Indenyl, die mit C₁-C₄-Alkyl substituiert sind; insbesonders bevorzugt steht R₁₀ für Cyclopentadienyl oder Indenyl, die mit Methyl substituiert sind.

Einige Beispiele für B sind Ferrocen und Fe(Indenyl)₂, und als Ferrocenderivate in Formel II Dimethyl-, Tetramethyl-, Hexamethyl-, Octamethyl- und Decamethylferrocen. Bevorzugte CT-Komplexe der Formel II sind solche aus 6,13-Dithio-5,7,12,14-tetracyanoiminpentacen und unsubstituiertem Ferrocen, Dimethyl-, Tetramethyl-, Hexamethyl-, Octamethyl- und Decamethylferrocen als B.

Ein anderer Gegenstand der Erfindung ist ein Verfahren zur Herstellung von CT-Komplexen der Formel II, das dadurch gekennzeichnet ist, dass man
a) wenn B unsubstituiertes Ferrocen, ein Ferrocenderivat oder Fulvalenderivat bedeutet, äquimolare Mengen des Ferrocens beziehungsweise eines Ferrocenderivates oder Fulvalenderivates B und eines 6,13-Dithiopentacenderivates der Formel I in einem inerten organischen Lösungsmittel umsetzt, oder
b) wenn B eine N-aromatische Verbindung bedeutet, 1 Äquivalent eines neutralen 5,10-Dihydrophenazinderivats oder dessen Iodsalz als B oder das Iodsalz einer N-aromatischen Verbindung B mit mindestens 1 Äquivalent eines 6,13-Dithiopentacenderivates der Formel I in einem inerten organischen Lösungsmittel umsetzt. Es kann aber zweckmässig sein, einen Überschuss des Dithiopentacenderivates zu verwenden.

Äquimolare Mengen bedeutet, dass man zur Bildung der 2:1-Komplexe etwa 1 Äquivalent des Ferrocens oder Ferrocenderivats B mit etwa 2 Äquivalenten des 6,13-Dithiopentacen-derivates der Formel I umsetzt, oder zur Bildung der 1:1-Komplexe etwa 1 Äquivalent des Fulvalenderivats der Formeln IIIa oder IIIb mit etwa 1 Äquivalent des 6,13-Dithiopentacenderivates der Formel I umsetzt. Die Ferrocenderivate und Fulvalenderivate sind bekannt, teilweise käuflich oder sie können nach allgemein bekannten Verfahren hergestellt werden.

Die N-aromatischen Verbindungen sind als Salze oder freie Basen bekannt, teilweise käuflich oder sie können nach allgemein bekannten Verfahren hergestellt werden. Reaktion zwischen der neutralen Base B und dem Alkyliodid ergibt das gewünschte Iodsalz. Die Herstellung von einigen von diesen Derivaten ist von L. Russell Melby in Can. J. Chem. Vol. 43, Seiten 1448 bis 1453 (1965) beschrieben.

Das erfindungsgemässe Verfahren zur Herstellung von CT-Komplexen wird vorteilhaft bei erhöhten Temperaturen durchgeführt, zum Beispiel bei Raumtemperatur bis 150°C. Zur Isolierung der erfindungsgemässen CT-Komplexe kann man das Reaktionsgemisch abkühlen und die ausgefallenen Kristalle abfiltrieren und durch auswaschen und/oder Umkristallisation reinigen.

Geeignete Lösungsmittel sind zum Beispiel unpolare, polare und aprotische Lösungsmittel, die allein oder in Mischungen aus mindestens zwei Lösungsmitteln verwendet werden können. Beispiele sind: Ether (Anisol, Dibutylether, Tetrahydrofuran, Dioxan, Ethylenglykolmonomethyl- oder -dimethylether, Ethylenglykolmonoethyl- oder -diethylether, Diethylenglykoldiethylether, Triethylenglykoldimethylether), halogenierte Kohlenwasserstoffe (Methylenchlorid, Chloroform, 1,2-Dichlorethan, 1,1,1-Trichlorethan, 1,1 ,2,2-Tetrachlorethan), Carbonsäureester und Lactone (Essigsäureethylester, Propionsäuremethylester, Benzoesäureethylester, 2-Methoxyethylacetat, γ-Butyrolacton, δ-Valerolacton, Pivalolacton), Carbonsäureamide und Lactame (N,N-Dimethylformamid, N,N-Diethylformamid, N,N-Dimethylacetamid, Tetramethylharnstoff, Hexamethylphosphorsäuretriamid, γ-Butyrolactam, ε-Caprolactam, N-Methylpyrrolidon, N-Acetylpyrrolidon, N-Methylcaprolactam), Ketone (Methylethylketon, Methylisobutylketon), Sulfoxide (Dimethylsulfoxid), Sulfone (Dimethylsulfon, Diethylsulfon, Trimethylensulfon, Tetramethylensulfon), tertiäre Amine (N-Methylpiperidin, N-Methylmorpholin) substituierte Benzole (Benzonitril, Chlorbenzol, o-Dichlorbenzol, 1,2,4-Trichlorbenzol, Nitrobenzol, Toluol, Xylol), Nitrile (Acetonitril, Propionitril) und aliphatische oder cycloaliphatische Kohlenwasserstoffe (Petrolether, Pentan, Hexan, Cyclohexan und Methylcyclohexan). Geeignet sind auch aromatisch-aliphatische Ether wie zum Beispiel Methyl- oder Ethylphenylether.

Bevorzugt sind polare Lösungsmittel, wenn B eine N-aromatische Verbindung bedeutet, weil die Iodsalze der Verbindungen B unter diesen Bedingungen besser löslich sind. Bevorzugte polare Lösungsmittel sind zum Beispiel Dimethylformamid und γ-Butyrolacton.

Die nach dem erfindungsgemässen Verfahren erhältlichen CT-Komplexe fallen in hohen Reinheiten an und brauchen nach der Filtration nur noch mit Lösungsmitteln gewaschen werden. Es handelt sich in der Regel um dunkelfarbige Kristalle mit nadelförmiger Kristallgestalt, die elektrische Leitfähigkeiten als Pressling von mehr als 10⁻⁴ S/cm aufweisen. Sie eignen sich hervorragend als elektrische Leiter. So können mit der Einarbeitung dieser CT-Komplexe in Kunststoffe je nach Art des CT-Komplexes und der verwendeten Menge elektrisch leitende oder antistatisch ausgerüstete Kunststoffe erhalten werden, wobei der CT-Komplex als Netzwerk der Kristallnadeln in der Kunststoffmatrix vorliegt. Je nach der Konzentration des CT-Komplexes in der Kunststoffmatrix können sehr feinmaschige Nadelnetzwerke erhalten werden.

Ein weiterer Gegenstand der Erfindung ist eine Zusammensetzung, enthaltend a) ein duroplastisches, thermoplastisches oder strukturell vernetztes Polymer und b) einen CT-Komplex der Formel II in Form eines Netzwerks aus Kristallnadeln in der Polymermatrix.

Die CT-Komplexe können in einer Menge von 0,01 bis 30 Gew.-%, bevorzugt 0,01 bis 20 Gew.-%, besonders bevorzugt 0,01 bis 10 Gew.-% und insbesondere bevorzugt 0,1 bis 5 Gew.-% enthalten sein, bezogen auf die Zusammensetzung.

Bei den thermoplastischen Polymeren kann es sich zum Beispiel um die folgenden Polymeren, Copolymeren bzw. Mischungen von diesen Polymeren handeln:
1. Polymere von Mono- und Diolefinen, beispielsweise Polypropylen, Polyisobutylen, Polybuten-1, Polymethylpenten-l, Polyisopren oder Polybutadien sowie Polymerisate von Cycloolefinen wie z.B. von Cyclopenten oder Norbornen; ferner Polyethylen (das gegebenenfalls vernetzt sein kann), z.B. Polyethylen hoher Dichte (HDPE), Polyethylen niederer Dichte (LDPE), lineares Polyethylen niederer Dichte (LLDPE).
2. Mischungen der unter 1) genannten Polymeren, z.B. Mischungen von Polypropylen mit Polyisobutylen, Polypropylen mit Polyethylen (z.B. PP/HDPE, PP/LDPE) und Mischungen verschiedener Polyethylentypen (z.B. LDPE/HDPE).
3. Copolymere von Mono- und Diolefinen untereinander oder mit anderen Vinylmonomeren, wie z.B. Ethylen-Propylen-Copolymere, lineares Polyethylen niederer Dichte (LLDPE) und Mischungen desselben mit Polyethylen niederer Dichte (LDPE), Propylen-Buten-1-Copolymere, Propylen-Isobutylen-Copolymere, Ethylen-Buten-1-Copolymere, Ethylen-Hexen-Copolymere, Ethylen-Methylpenten-Copolymere, Ethylen-Hepten-Copolymere, Ethylen-Octen-Copolymere, Propylen-Butadien-Copolymere, Isobutylen-Isopren-Copolymere, Ethylen-Alkylacrylat-Copolymere, Ethylen-Alkylmethacrylat-Copolymere, Ethylen-Vinylacetat-Copolymere oder Ethylen-Acrylsäure-Copolymere und deren Salze (Ionomere), sowie Terpolymere von Ethylen mit Propylen und einem Dien, wie Hexadien, Dicyclopentadien oder Ethylidennorbornen; ferner Mischungen solcher Copolymere untereinander und mit unter 1) genannten Polymeren, z.B. Polypropylen/Ethylen-Propylen-Copolymere, LDPE/Ethylen-Vinylacetat-Copolymere, LDPE/Ethylen-Acrylsäure-Copolymere, LLDPE/Ethylen-Vinylacetat-Copolymere und LLDPE/Ethylen-Acrylsäure-Copolymere.
   3a. Kohlenwasserstoffharze (z.B. C₅-C₉) inklusive hydrierte Modifikationen davon (z.B. Klebrigmacherharze).
4. Polystyrol, Poly-(p-methylstyrol), Poly-(α-methylstyrol).
5. Copolymere von Styrol oder α-Methylstyrol mit Dienen oder Acrylderivaten, wie z.B. Styrol-Butadien, Styrol-Acrylnitril, Styrol-Alkylmethacrylat, Styrol-Butadien-Alkylacrylat, Styrol-Maleinsäureanhydrid, Styrol-Acrylnitril-Methylacrylat; Mischungen von hoher Schlagzähigkeit aus Styrol-Copolymeren und einem anderen Polymer, wie z.B. einem Polyacrylat, einem Dien-Polymeren oder einem Ethylen-Propylen-Dien-Terpolymeren; sowie Block-Copolymere des Styrols, wie z.B. Styrol-Butadien-Styrol, Styrol-Isopren-Styrol, Styrol-Ethylen/Butylen-Styrol oder Styrol-Ethylen/Propylen-Styrol.
6. Pfropfcopolymere von Styrol oder α-Methylstyrol, wie z.B. Styrol auf Polybutadien, Styrol auf Polybutadien-Styrol- oder Polybutadien-Acrylnitril-Copolymere, Styrol und Acrylnitril (bzw. Methacrylnitril) auf Polybutadien; Styrol, Acrylnitril und Methylmethacrylat auf Polybutadien; Styrol und Maleinsäureanhydrid auf Polybutadien; Styrol, Acrylnitril und Maleinsäureanhydrid oder Maleinsäureimid auf Polybutadien; Styrol und Maleinsäureimid auf Polybutadien, Styrol und Alkylacrylate bzw. Alkylmethacrylate auf Polybutadien, Styrol und Acrylnitril auf Ethylen-Propylen-Dien-Terpolymeren, Styrol und Acrylnitril auf Polyalkylacrylaten oder Polyalkylmethacrylaten, Styrol und Acrylnitril auf Acrylat-Butadien-Copolymeren, sowie deren Mischungen mit den unter 5) genannten Copolymeren, wie sie z.B. als sogenannte ABS-, MBS-, ASA- oder AES-Polymere bekannt sind.
7. Halogenhaltige Polymere, wie z.B. Polychloropren, Chlorkautschuk, chloriertes oder chlorsulfoniertes Polyethylen, Copolymere von Ethylen und chloriertem Ethylen, Epichlorhydrinhomo- und -copolymere, insbesondere Polymere aus halogenhaltigen Vinylverbindungen, wie z.B. Polyvinylchlorid, Polyvinylidenchlorid, Polyvinylfluorid, Polyvinylidenfluorid; sowie deren Copolymere, wie Vinylchlorid-Vinylidenchlorid, Vinylchlorid-Vinylacetat oder Vinylidenchlorid-Vinylacetat.
8. Polymere, die sich von α,β-ungesättigten Säuren und deren Derivaten ableiten, wie Polyacrylate und Polymethacrylate, Polyacrylamide und Polyacrylnitrile.
9. Copolymere der unter 8) genannten Monomeren untereinander oder mit anderen ungesättigten Monomeren, wie z.B. Acrylnitril-Butadien-Copolymere, Acrylnitril-Alkylacrylat-Copolymere, Acrylnitril-Alkoxyalkylacrylat-Copolymere, Acrylnitril-Vinylhalogenid-Copolymere oder Acrylnitril-Alkylmethacrylat-Butadien-Terpolymere.
10. Polymere, die sich von ungesättigten Alkoholen und Aminen bzw. deren Acylderivaten oder Acetalen ableiten, wie Polyvinylalkohol, Polyvinylacetat, -stearat, -benzoat, -maleat, Polyvinylbutyral, Polyallylphthalat, Polyallylmelamin; sowie deren Copolymere mit in Punkt 1 genannten Olefinen.
11. Homo- und Copolymere von cyclischen Ethern, wie Polyalkylenglykole, Polyethylenoxyd, Polypropylenoxyd oder deren Copolymere mit Bisglycidylethern.
12. Polyacetale, wie Polyoxymethylen, sowie solche Polyoxymethylene, die Comonomere, wie z.B. Ethylenoxid, enthalten; Polyacetale, die mit thermoplastischen Polyurethanen, Acrylaten oder MBS modifiziert sind.
13. Polyphenylenoxide und -sulfide und deren Mischungen mit Styrolpolymeren oder Polyamiden.
14. Polyurethane, die sich von Polyethern, Polyestern und Polybutadienen mit endständigen Hydroxylgruppen einerseits und aliphatischen oder aromatischen Polyisocyanaten andererseits ableiten, sowie deren Vorprodukte.
15. Polyamide und Copolyamide, die sich von Diaminen und Dicarbonsäuren und/oder von Aminocarbonsäuren oder den entsprechenden Lactamen ableiten, wie Polyamid 4, Polyamid 6, Polyamid 6/6, 6/10, 6/9, 6/12, 4/6, Polyamid 11, Polyamid 12, aromatische Polyamide ausgehend von m-Xylylendiamin und Adipinsäure; Polyamide, hergestellt aus Hexamethylendiamin und Iso- und/oder Terephthalsäure und gegebenenfalls einem Elastomer als Modifikator, z.B. Poly-2,4,4-trimethylhexamethylenterephthalamid, Poly-m-phenylen-isophthalamid. Block-Copolymere der vorstehend genannten Polyamide mit Polyolefinen, Olefin-Copolymeren, Ionomeren oder chemisch gebundenen oder gepfropften Elastomeren; oder mit Polyethern, wie z.B. mit Polyethylenglykol, Polypropylenglykol oder Polytetramethylenglykol. Ferner mit EPDM oder ABS modifizierte Polyamide oder Copolyamide; sowie während der Verarbeitung kondensierte Polyamide ("RIM-Polyamidsysteme").
16. Polyharnstoffe, Polyimide, Polyamid-imide und Polybenzimidazole.
17. Polyester, die sich von Dicarbonsäuren und Dialkoholen und/oder von Hydroxycarbonsäuren oder den entsprechenden Lactonen ableiten, wie Polyethylenterephthalat, Polybutylenterephthalat, Poly-1,4-dimethylolcyclohexanterephthalat, Polyhydroxybenzoate, sowie Block-Polyether-ester, die sich von Polyethern mit Hydroxylendgruppen ableiten; ferner mit Polycarbonaten oder MBS modifizierte Polyester.
18. Polycarbonate und Polyestercarbonate.
19. Polysulfone, Polyethersulfone und Polyetherketone.
20. Polyether aus Diglycidylverbindungen, zum Beispiel Diglycidylethern und Diolen, zum Beispiel aus Bisphenol-A-Diglycidylether und Bisphenol-A.
21. Natürliche Polymere, wie Cellulose, Naturkautschuk, Gelatine, sowie deren polymerhomolog chemisch abgewandelte Derivate, wie Celluloseacetate, -propionate und -butyrate, bzw. die Celluloseether, wie Methylcellulose; sowie Kolophoniumharze und Derivate.
22. Mischungen (Polyblends) der vorgenannten Polymeren, wie z.B. PP/EPDM, Polyamid/EPDM oder ABS, PVC/EVA, PVC/ABS, PVC/MBS, PC/ABS, PBTP/ABS, PC/ASA, PC/PBT, PVC/CPE, PVC/Acrylate, POM/thermoplastisches PUR, PC/thermoplastisches PUR, POM/Acrylat, POM/MBS, PPO/HIPS, PPO/PA 6.6 und Copolymere, PA/HDPE, PA/PP, PA/PPO.

Bevorzugte Thermoplaste sind Polyolefine, Polystyrol, Polyvinylchlorid, Polyvinylidenchlorid, Polyvinylidenfluorid, Polyacrylate, Polymethacrylate, Polyamide, Polyester, Polycarbonate, aromatische Polysulfone, aromatische Polyether, aromatische Polyethersulfone, Polyimide und Polyvinylcarbazol.

Bei den Duroplasten und strukturell vernetzten Polymeren kann es sich zum Beispiel um folgende Polymere handeln:
1. Vernetzte Polymere, die sich von Aldehyden einerseits und Phenolen, Harnstoff oder Melamin andererseits ableiten, wie Phenol-Formaldehyd-, Harnstoff-Formaldehyd- und Melamin-Formaldehydharze.
2. Trocknende und nicht-trocknende Alkydharze.
3. Ungesättigte Polyesterharze, die sich von Copolyestern gesättigter und ungesättigter Dicarbonsäuren mit mehrwertigen Alkoholen, sowie Vinylverbindungen als Vernetzungsmittel ableiten, wie auch deren halogenhaltige, schwerbrennbare Modifikationen.
4. Vernetzbare Acrylharze, die sich von substituierten Acrylsäureestern ableiten, wie z.B. von Epoxyacrylaten, Urethan-acrylaten oder Polyester-acrylaten.
5. Alkydharze, Polyesterharze und Acrylatharze, die mit Melaminharzen, Harnstoffharzen, Polyisocyanaten oder Epoxidharzen vernetzt sind.
6. Kautschuk auf der Basis von vernetzten Polydienen, zum Beispiel Butadien oder Isopren; Silikonkautschuk.
7. Epoxidharze, die sich von Polyepoxiden ableiten, z.B. von Bisglycidylethern von Polyolen oder von cycloaliphatischen Diepoxiden, und die gegebenenfalls einen Härter als Vernetzungsmittel enthalten, oder die unter Verwendung von Härtungsbeschleunigern thermisch oder durch Einwirkung von Strahlung vernetzt sind.

Unter den vernetzten Polymeren sind vernetzte Epoxidharze bevorzugt, denen als Polyepoxide bevorzugt Glycidylverbindungen mit durchschnittlich zwei Epoxidgruppen im Molekül zu Grunde liegen. Als Glycidylverbindungen kommen vor allem solche mit zwei an ein Heteroatom (z.B. Schwefel, vorzugsweise Sauerstoff oder Stickstoff) gebundenen Glycidylgruppen, β-Methylglycidylgruppen oder 2,3-Epoxycyclopentylgruppen in Frage; genannt seien insbesondere Bis-(2,3-epoxycyclopentyl)-ether; Diglycidylether von mehrwertigen aliphatischen Alkoholen, wie 1,4-Butandiol, oder Polyalkylenglykolen, wie Polypropylenglykole; Diglycidylether von cycloaliphatischen Polyolen, wie 2,2-Bis-(4-hydroxycyclohexyl)-propan; Diglycidylether von mehrwertigen Phenolen, wie Resorcin, Bis-(p-hydroxyphenyl)-methan, 2,2-Bis-(p-hydroxyphenyl)-propan (=Diomethan), 2,2-Bis-(4'-hydroxy-3',5'-dibromphenyl)-propan, 1,3-Di-(p-hydroxyphenyl)-ethan; Di-(β-methylglycidyl)-ether der oben angeführten zweiwertigen Alkohole oder zweiwertigen Phenole; Diglycidylester von Dicarbonsäuren, wie Phthalsäure, Terephthalsäure, Δ₄-Tetrahydrophthalsäure und Hexahydrophthalsäure; N,N-Diglycidylderivate von primären Aminen und Amiden und heterocyclischen, zwei N-Atome enthaltenden Stickstoffbasen, und N,N'-Diglycidylderivate von disekundären Diamiden und Diaminen, wie N,N-Diglycidylanilin, N,N-Diglycidyltoluidin, N,N-Diglycidyl-p-aminophenyl-methyl-ether, N,N'-Dimethyl-N,N-diglycidyl-bis-(p-aminophenyl)-methan; N',N''-Diglycidyl-N-phenyl-isocyanurat; N,N-Diglycidylethylenharnstoff; N,N'-Diglycidyl-5,5-dimethyl-hydantoin, N,N-Diglycidyl-5-isopropyl-hydantoin, N,N-Methylen-bis-(N',N'-diglycidyl-5,5-dimethylhydantoin), 1,3-Bis-(N-glycidyl-5,5-dimethylhydantoin)-2-hydroxypropan; N,N'-Diglycidyl5,5-dimethyl-6-isopropyl-5,6-dihydro-uracil, Triglycidylisocyanurat.

Eine bevorzugte Gruppe von Epoxidharzen sind glycidylierte Novolake, Hydantoine, Aminophenole, Bisphenole und aromatische Diamine oder cycloaliphatische Epoxidverbindungen. Besonders bevorzugte Epoxidharze sind glycidylierte Kresolnovolake, Bisphenol-A- und Bisphenol-F-diglycidylether, Hydantoin-N,N'-bisglycid, p-Aminophenoltriglycid, Diaminodiphenylmethantetraglycid, Vinylcyclohexendioxid, 3,4-Epoxycyclohexylmethyl-3,4-epoxycyclohexancarboxylat oder Mischungen hiervon.

Geeignet sind auch vorreagierte Addukte solcher Epoxidverbindungen mit Epoxidhärtern, zum Beispiel ein Addukt aus Bisphenol-A-diglycidylether und Bisphenol-A, oder mit Oligoestern mit zwei terminalen Carboxylgruppen und Epoxiden vorreagierte Addukte.

Als Härter für Epoxidharze kommen säure oder basische Verbindungen in Frage. Als geeignete Härter seien zum Beispiel genannt: mehrwertige Phenole (Resorcin, 2,2-Bis-(4-hydroxyphenyl)-propan) oder Phenol-Formaldehyd-Harze; mehrbasische Carbonsäuren und ihre Anhydride, z. B. Phthalsäureanhydrid, Tetrahydrophthalsäureanhydrid, Hexahydrophthalsäureanhydrid, 4-Methylhexahydrophthalsäureanhydrid, 3,6-Endomethylen-tetrahydrophthalsäreanhydrid, 4-Methyl-3,6-endomethylen-tetrahydrophthalsäureanhydrid (Methylnadicanhydrid), 3,4,5,6,7,7-Hexachlor-endomethylen-tetrahydrophthalsäureanhydrid, Bernsteinsäureanhydrid, Adipinsäureanhydrid, Trimethyladipinsäureanhydrid, Sebacinsäureanhydrid, Maleinsäureanhydrid, Dodecylbernsteinsäureanhydrid, Pyromellitsäuredianhydrid, Trimellitsäureanhydrid, Benzophenontetracarbonsäuredianhydrid, oder Gemische solcher Anhydride.

Eine bevorzugte Gruppe von Härtern sind Novolake und Polycarbonsäureanhydride.

Die Epoxidharze können auch zusätzlich mit Härtungsbeschleunigern oder nur mit thermischen Härtungskatalysatoren gehärtet werden. Beispiele sind 3-Ethyl-4-methylimidazol, Triamylammoniumphenolat; Mono- oder Polyphenole (Phenol, Diomenthan, Salicylsäure); Bortrifluorid und seine Komplexe mit organischen Verbindungen wie z.B. Bortrifluorid-Etherkomplexe und BortrifluoridAmin-Komplexe (BF₃-Monoethylamin-Komplex); Phosphorsäure und Triphenylphosphit.

Härtungsbeschleuniger und Katalysatoren werden üblicherweise in einer Menge von 0,1 bis 10 Gew.-% zugegeben, bezogen auf das Epoxidharz. Härter für Epoxidharze werden im allgemeinen in äquimolaren Mengen verwendet, bezogen auf die Epoxidgruppen und funktionellen Gruppen eines Härters.

Der erfindungsgemässen Zusammensetzung können weitere Additive zur Verbesserung der Verarbeitungseigenschaften, der mechanischen, elektrischen und thermischen Eigenschaften, der Oberflächeneigenschaften und der Lichtstabilität einverleibt sein, zum Beispiel feinteilige Füllstoffe, Verstärkerfüllstoffe, Weichmacher, Gleit- und Entformungsmittel, Haftvermittler, Antistatika, Antioxidantien, Wärme- und Lichtstabilisatoren, Pigmente und Farbstoffe.

Eine bevorzugte Ausführungsform der erfindungsgemässen Zusammensetzung ist dadurch gekennzeichnet, dass sie als Formkörper, Filme, Folien, Fasern, oder als Beschichtungen auf mindestens einer Oberfläche eines Substrats ausgebildet ist.

Ein weiterer Gegenstand der Erfindung ist ein Verfahren zur Herstellung von erfindungsgemässen Zusammensetzungen, das dadurch gekennzeichnet ist, dass man (a) einen CT-Komplex der Formel II einem thermoplastischen Kunststoff einverleibt, (b) einen CT-Komplex der Formel II mindestens einer Komponente eines duroplastischen oder strukturell vernetzbaren Kunststoffs einverleibt und dann die Mischung gegebenenfalls zusammen mit weiteren Komponenten zu einem duroplastischen oder strukturell vernetzten Kunststoff polymerisiert, oder (c) eine Verbindung der Formel I oder Ferrocen oder ein Ferrocenderivat B, ein Fulvalenderivat der Formeln IIIa oder IIIb als B, ein 5,10-Dihydrophenazinderivat oder dessen Iodsalz als B oder das Iodsalz einer N-aromatischen Verbindung B zusammen mit einem thermoplastischen Kunststoff oder mindestens einer Komponente eines duroplastischen oder strukturell vernetzbaren Kunststoffs in einem organischen Lösungsmittel löst, diese Lösung gegebenenfalls zusammen mit weiteren Komponenten für einen duroplastischen oder strukturell vernetzbaren Kunststoff mit einer Lösung Ferrocens oder eines Ferrocenderivats B, eines Fulvalenderivats der Formeln IIIa oder IIIb als B, eines 5,10-Dihydrophenazindenvats oder dessen Iodsalz als B oder eines Iodsalzes einer N-aromatischen Verbindung B beziehungsweise einer Verbindung der Formel I vermischt, das Lösungsmittel entfernt und härtbare Mischungen zu einem duroplastischen oder strukturell vernetzten Kunststoff polymerisiert. Das Herstellungsverfahren kann mit einer Formgebung verbunden sein.

Die Herstellung der erfindungsgemässen Zusammensetzung kann nach in der Kunststofftechnik bekannten Verfahren erfolgen. Bei Formgebungsverfahren für Polymere, zum Beispiel Giessen, Pressverfahren, Spritzgiessen und Extrusion, kann der CT-Komplex selbst unter Bildung von Suspensionen einem Thermoplasten oder mindestens einem Ausgangsstoff für Duroplaste, oder getrennt je einem Ausgangsstoff (zum Beispiel dem Epoxidharz und dem Härter) unter Bildung von Lösungen oder Suspensionen zugegeben werden, wobei nach der Formgebung der CT-Komplex während des Abkühlens in Form von Nadeln auskristallisiert beziehungsweise ausfällt, und die Nadeln ein Netzwerk in einer Polymermatrix bilden.

In einer besonders bevorzugten Ausführungsform ist die erfindungsgemässe Zusammensetzung als Film oder Folie oder als Beschichtung auf mindestens einer Oberfläche eines Substrats ausgebildet. Zur Herstellung solcher Ausführungsformen wird zum Beispiel ein thermoplastisches Polymer oder mindestens ein Ausgangsprodukt für einen Duroplasten oder ein strukturell vernetztes Polymer in einem inerten Lösungsmittel zusammen mit einem CT-Komplex der Formel II suspendiert und /oder gelöst, oder zusammen mit einer Verbindung der Formel I oder Ferrocen oder einem Ferrocenderivat B, einem Fulvalenderivat B, oder einem Iodsalz einer N-aromatischen Verbindung B gelöst und dann mit einer Lösung Ferrocens oder des Ferrocenderivats B, des Fulvalenderivats B, oder des N-aromatischen Iodsalzes B beziehungsweise einer Verbindung der Formel I versetzt und vermischt, danach mittels bekannter Beschichtungstechniken auf ein gegebenenfalls vorerwärmtes Substrat aufgetragen und dann unter Erwärmen das Lösungsmittel entfernt, wobei vernetzbare Mischungen dann noch ausgehärtet werden können. Freitragende Filme und Folien werden durch Ablösen vom Substrat oder mittels Extrusionsverfahren erhalten.

Geeignete Substrate sind zum Beispiel Glas, Metalle, Kunststoffe, mineralische und keramische Werkstoffe, Holz und Papier. Die Substrate können beliebige äussere Formen aufweisen; es kann sich zum Beispiel um Formkörper, Fäden, Fasern, Gewebe, Stangen, Rohre, Bänder, Folien, Platten, Walzen oder Gehäuse handeln.

Geeignete Beschichtungsverfahren sind zum Beispiel Streichen, Walzen, Rakeln, Giessen, Schleudergiessen, Vorhanggiessen und Sprayen. Besonders bevorzugte Verfahren sind Sprayverfahren, weil zum einen sehr dünne und gleichmässige Schichten mit im wesentlichen isotropen, sehr feinmaschigen und homogenen Netzwerken aus Kristallnadeln der CT-Komplexe erhältlich sind, und zum anderen die Grösse der Kristallnadeln und die Maschenweite der Netzwerke durch die Tröpfchengrösse kontrolliert werden kann, selbst wenn man Suspensionen sprüht.

Geeignete inerte Lösungsmittel für Polymere beziehungsweise Ausgangsprodukte für Polymere sind zum Beispiel polare und bevorzugt aprotische Lösungsmittel, die alleine oder in Mischungen aus mindestens zwei Lösungsmitteln verwendet werden können. Beispiele sind: Ether (Anisol, Dibutylether, Tetrahydrofuran, Dioxan, Ethylenglykolmonomethyl- oder -dimethylether, Ethylenglykolmonoethyl- oder -diethylether, Diethylenglykoldiethylether, Triethylenglykoldimethylether), halogenierte Kohlenwasserstoffe (Methylenchlorid, Chloroform, 1,2-Dichlorethan, 1,1,1-Trichlorethan, 1,1,2,2-Tetrachlorethan), Carbonsäureester und Lactone (Essigsäureethylester, Propionsäuremethylester, Benzoesäureethylester, 2Methoxyethylacetat, γ-Butyrolacton, δ-Valerolacton, Pivalolacton), Carbonsäureamide und Lactame (N,N-Dimethylformamid, N,N-Diethylformamid, N,N-Dimethylacetamid, Tetramethylharnstoff, Hexamethylphosphorsäuretriamid, γ-Butyrolactam, ε-Caprolactam, N-Methylpyrrolidon, N-Acetylpyrrolidon, N-Methylcaprolactam), Ketone (Methylethylketon, Methylisobutylketon), Sulfoxide (Dimethylsulfoxid), Sulfone (Dimethylsulfon, Diethylsulfon, Trimethylensulfon, Tetramethylensulfon), tertiäre Amine (N-Methylpiperidin, N-Methylmorpholin) substituierte Benzole (Benzonitril, Chlorbenzol, o-Dichlorbenzol, 1,2,4-Trichlorbenzol, Nitrobenzol, Toluol, Xylol) und Nitrile (Acetonitril, Propionitril). Geeignet sind auch aromatisch-aliphatische Ether wie zum Beispiel Methyl- oder Ethylphenylether. Geeignete Lösungsmittel für die Verbindungen der Formel II und die N-aromatischen Verbindungen/Iodsalze B sind zuvor genannt worden.

Die Beschichtungsverfahren können zum Beispiel so durchgeführt werden, dass man die einzelnen Bestandteile getrennt löst und erst vor dem gewählten Beschichtungsverfahren vereinigt. Man kann aber auch die Bestandteile in zwei Lösungen, zum Beispiel Polymerlösung und Ferrocen oder Ferrocenderivat B, Fulvalenderivat B oder N-aromatisches Iodsalz B oder einer Verbindung der Formel I und Lösung einer Verbindung der Formel I oder Ferrocens oder eines Ferrocenderivats B, Fulvalenderivats B oder eines N-aromatischen Iodsalzes B gegebenenfalls zusammen mit einem Polymer herstellen, oder alle Bestandteile in einer Lösung vereinigen. Im letzten Fall können die CT-Komplexe schon vor der Beschichtung auskristallisieren, was aber praktisch keinen Einfluss auf die gewünschte Qualität der Beschichtung hat.

Die Lösungen werden zweckmässig erwarmt, zum Beispiel auf 30 bis 200 °C. Es ist vorteilhaft, auch das Substrat zu erwärmen, um die Entfernung des Lösungsmittels zu beschleunigen, die im allgemeinen bei Temperaturen von 50 bis 150 °C, bevorzugt 50 bis 100 °C vorgenommen wird, bis die Beschichtung trocken ist. Sofern die Beschichtungen zu frei tragenden Filmen oder Folien abgelöst werden sollen, kann das Substrat vor der Beschichtung mit Antihaftmitteln behandelt werden.

In einer Herstellvariante für Beschichtungen kann man auch die erfindungsgemässen CT-Komplexe, die als Kristallnadeln ausgebildet sind, in einer Lösung eines Polymeren oder Ausgangsstoffen für Duroplaste suspendieren, dann ein Substrat beschichten und danach das Lösungsmittel entfernen und gegebenenfalls zur Bildung der Duroplaste aushärten. Ferner ist es möglich, pulvrige Trockenmischungen aus Polymerpulvern oder festen Ausgangsstoffen für Duroplaste und den CT-Komplexen herzustellen und diese in gegebenenfalls elektrostatischen Beschichtungsverfahren zu Schichten auf Substraten zu verarbeiten. Auch bei diesen Varianten werden Netzwerke von Kristallnadeln in einer Polymermatrix erhalten.

Es ist auch möglich, reine Schichten von Netzwerken aus Kristallnadeln der CT-Komplexe auf einem Substrat herzustellen, indem man zum Beispiel Lösungen oder Suspensionen der CT-Komplexe in einem Lösungsmittel auf einem Substrat aufbringt und danach das Lösungsmittel verdampft. Solche Schichten können zur Erhöhung der elektrischen Leitfähigkeit elektrochemisch metallisiert werden, zum Beispiel mit Cu, Pt oder Pd. Es kann zweckmässig sein, solche reine Schichten mit einer Schutzschicht aus einem Polymer zu beschichten oder die reinen Schichten nachträglich mit einem Polymer zu umhüllen.

Die Schichtdicken können in einem breiten Rahmen variieren, je nach Wahl des Beschichtungsverfahrens. Mit Sprayverfahren können sehr dünne Schichten erhalten werden, während man mit Streich- und Giessverfahren auch dicke Schichten erzielen kann. Die Schichtdicken können zum Beispiel 0,01 bis 5000 µm, bevorzugt 0,1 bis 1000 µm und besonders bevorzugt 0,1 bis 500 µm betragen.

Die erfindungsgemässe Zusammensetzung ist je nach Wahl des Polymers opak oder transparent und sie weist hervorragende elektrische Eigenschaften auf. So weisen die Beschichtungen und Formkörper überraschend eine ausgezeichnete Entladungsfähigkeit auf, die für heterogene Materialien sonst schwierig oder gar nicht zu erreichen ist. Die Zusammensetzung eignet sich daher besonders zur Verwendung als antistatisch ausgerüstete Formteile für die elektrostatische Abschirmung von Bauteilen oder zur Verwendung als antistatisch ausgerüstete Formkörper. Die hohen elektrischen Leitfähigkeiten ermöglichen ferner die Verwendung als elektrische Leiter, zum Beispiel als Elektroden für Displayelemente oder elektronische Bauteile sowie als Ladungsspeicher in Kondensatoren. Die Zusammensetzungen weisen auch hervorragende mechanische Festigkeiten und mechanische Gebrauchseigenschaften auf. Die Zusammensetzungen können auch bei vergleichsweise niedrigen Temperaturen hergestellt werden und bieten zudem den Vorteil, keine oder nur eine unwesentliche Korrosion bei metallischen Maschinenteilen zu verursachen. Ferner weisen sie gute Stabilitäten gegenüber dem Einfluss von Wärme und/oder Feuchtigkeit auf.

Weitere Gegenstände der Erfindung sind die Verwendung der erfindungsgemässen Verbindungen der Formel I als Elektronenakzeptoren; die Verwendung der erfindungsgemässen Verbindungen der Formel I zur Herstellung von Charge-Transfer Komplexen; die Verwendung der erfindungsgemässen Charge-Transfer Komplexe der Formel II als elektrische Leiter; die Verwendung der erfindungsgemässen Zusammensetzung als antistatisch ausgerüstete Formteile für die elektrostatische Abschirmung von Bauteilen oder als antistatisch ausgerüstete Formkörper; die Verwendung der erfindungsgemässen Zusammensetzung als elektrische Leiter; die Verwendung der erfindungsgemässen Zusammensetzung als Elektrodenmaterial und die Verwendung der erfindungsgemässen Zusammensetzung in Form von Filmen oder Folien als Ladungsträger in Kondensatoren.

Dinaphtho[2,3-b;2',3'-e][1,4]dithiin-5,7,12,14-tetracyanoimin (6,13-Dithio-5,7,12,14-tetracyanoiminpentacen) weist vier reversible Reduktionspotentiale auf, gemessen in DMF, 0,1 mol (C₄H₉)₄NBF₄ um 50 mV/s: +0,322, +0,122, -0,390 und -0,600 V bezogen auf die Standardkalomelelektrode (SKE). Dadurch ist 6,13-Dithiotetracyanoiminpentacen ein besonders starker Elektronenakzeptor im Vergleich mit TCNQ oder 5,7,12,14-Tetracyanoiminpentacen, und kann CT-Komplexe mit einem grösseren Umfang von Donatoren bilden. Dinaphtho[2,3-b;2',3'-e][1,4]dithiin-5,7,12,14-tetracyanoimin an sich weist bei Raumtemperatur eine elektrische Leitfähigkeit von 3,3 · 10⁻⁷ S/cm als Pressling auf.

Die nachfolgenden Beispiele erläutern die Erfindung näher.

### A) Herstellungsbeispiele

### Beispiel A1: Herstellung von 6,13-Dithio-5,7,l2,14-tetracyanoiminpentacen(Dinaphtho[2,3-b;2',3'-e][1,4]dithiin-5,7,12,14-tetracyanoimin)

Zu einer Suspension von 0,5 g (1,33 mMol) DNDT in 50 ml Dichlormethan unter Argon werden 2,52 g (13,28 mMol) TiCl₄ zugetropft. Nach 1 h Rühren werden 2,97 g (15,94 mMol) Bis(trimethylsilylcarbodiimide) ([(CH₃)₃SiN]₂C) zugegeben. Das Reaktionsgemisch wird 20 h unter Argon unter Rückfluss gekocht und ergibt eine dunkel-rote Suspension. Nach Abkühlung giesst man das Gemisch auf 50 g Eis und extrahiert mit 100 ml Dichlormethan. Die organische Schicht wird abgetrennt und mit Na₂SO₄ getrocknet und filtriert. Nach Abdampfung des Lösungsmittels ergibt sich ein dunkel-grüner Rückstand (0,08 g). Die wässrige Suspension wird filtriert und gibt 0,58 g eines dunkelgrünen Rückstands. Die zwei Portionen werden kombiniert und unter Rückfluss in 200 ml Anisol 15 min gekocht. Die dunkel-rote Lösung wird filtriert und bis Raumtemperatur abkühlen gelassen, während es feine schwarze Nadeln bilden. Die Nadeln werden filtriert und mit Pentan gewaschen. Man erhält 370 mg der Titelverbindung. Elementaranalyse gefunden (berechnet) für C₂₄H₈N₈S₂·1,2 Anisol: C 64,31;(64,61); H 3,02 (2,95); N 18,44 (18,60); S 10,93 (10,60).

### Beispiel A2: Herstellung von 2,3,9,10-Tetramethyl-dinaphtho[2,3-b;2',3'-e][1,4]dithiin-5,7,12,14-tetracyanoimin

Zu einer Suspension von 0,58 g (1,33 mMol) 2,3,9,10-Tetramethyl-dinaphtho-[2,3-b;2',3'-e][1,4]dithiin-5,7,12,14-tetron in 50 ml Dichlormethan unter Argon werden 2,52 g (13,28 mMol) TiCl₄ zugetropft. Nach 15 min Rühren werden 2,97 g (15,94 mMol) [(CH₃)₃SiN]₂C zugegeben. Das Reaktionsgemisch wird 16 h unter Argon unter Rückfluss gekocht. Nach Abkühlung giesst man das Gemisch auf 100 ml Eis und extrahiert mit 100 ml Dichlormethan. Die organische Schicht wird abgetrennt und mit Na₂SO₄ getrocknet und filtriert. Nach Abdampfung des Lösungsmittels ergibt sich ein dunkel-grüner Rückstand (0,4 g). Die wässrige Suspension wird filtriert und gibt 0,35 g eines dunkelgrünen Rückstands. Die zwei festen Portionen werden kombiniert und unter Rückfluss in 300 ml Anisol 15 min gelöst. Die dunkel-rote Lösung wird filtriert und bis Raumtemperatur abkühlen gelassen. Diethyläther wird zugegeben, um schwarze Nadeln ausfallen zu lassen. Die Nadeln werden filtriert und mit Pentan gewaschen. Man erhält 0,25 g der Titelverbindung. Elementaranalyse gefunden (berechnet) für C₂₈H₁₆N₈S₂·0,5 Anisol: C 63,60 (64,92); H 3,56 (3,47); N 18,30 (19,23); S 11,07 (11,00).

### Beispiel A3: Herstellung von 2,9-Dimethyl-dinaphtho[2,3-b;2',3'-e][1,4]dithiin-5,7,12,14-tetracyanoimin

Zu einer Suspension von 1,88 g (4,65 mMol) 2,9-Dimethyl-dinaphtho[2,3-b;2',3'-e]-[1,4]dithiin-5,7,12,14-tetron in 100 ml Dichlormethan unter Argon werden 8,82 g (46,48 mMol) TiCl₄ zugegeben. Nach 15 min Rühren werden 10,40 g (55,78 mMol) [(CH₃)₃SiN]₂C zugegeben. Das Reaktionsgemisch wird 16 h unter Argon unter Rückfluss gekocht. Nach Abkühlung giesst man das Gemisch auf 100 ml Eis und extrahiert mit 100 ml Dichlormethan. Die organische Schicht wird abgetrennt und mit Na₂SO₄ getrocknet und filtriert. Nach Abdampfung des Lösungsmittels ergibt sich ein dunkel-grüner Rückstand (1,66 g). Der Rückstand wird in 300 ml Trichlorbenzol gelöst, filtriert und bis Raumtemperatur abkühlen gelassen. Diethyläther wird zugegeben, um braune Nadeln ausfallen zu lassen. Die Nadeln werden filtriert und mit Pentan gewaschen. Man erhält 0,75 g der Titelverbindung. Elementaranalyse gefunden (berechnet) für C₂₆H₁₂N_{S}S₂·0,7 Äther: C 61,94 (62,62); H 3,20 (3,47); N 19,75 (20,28); S 11,60 (11,60). Wechselspannung in 0,1 M Bu₄NBF₄ Lösung in Dichlormethan gemessen um 50 mV/s: +0,327; +0,075; -0,375; -0,615 V bezogen auf die SKE.

### B) Herstellung von CT-Komplexen

### Beispiel B1: Herstellung eines CT-Komplexes aus Hexamethylferrocen und 6,13-Dithio-5,7,12,14-tetracyanoiminpentacen

Zu einer 150 °C heissen Lösung von 100 mg (0,212 mMol) 6,13-Dithio-5,7,12,14-tetracyanoiminpentacen in 50 ml Anisol wird eine gleich warme Lösung von 28,6 mg (0,106 mMol) Hexamethylferrocen in 5 ml Anisol gegeben. Man lässt die resultierende Lösung abkühlen und der CT-Komplexe wird abfiltriert. Danach werden die ausgefallenen dunkel-braunen Kristallnadeln mit Pentan gewaschen und dann im Hochvakuum getrocknet. Man erhält 66 mg der Titelverbindung, deren elektrische Leitfähigkeit (gemessen nach der Vierpunktmethode an einem Pressling) 0,35 S/cm beträgt; Smp. 214 °C. Elementaranalyse gefunden (berechnet) für C₆₄H₃₈N₁₆S₄Fe: C 63,05 (63,26); H 3,34 (3,15); N 18,08 (18,44); Fe 4,08 (4,60); S 10,69 (10,55).

### Beispiel B2: Herstellung eines CT-Komplexes aus Dimethylferrocen und 6,13-Dithio-5,7,12,14-tetracyanoiminpentacen

Zu einer 150 °C heissen Lösung von 100 mg (0,212 mMol) 6,13-Dithio-5,7,12,14-tetracyanoiminpentacen in 50 ml Anisol wird eine gleich warme Lösung von 23 mg (0,106 mMol) Dimethylferrocen in 5 ml Anisol gegeben. Man lässt die resultierende Lösung abkühlen und der CT-Komplexe wird abfiltriert. Danach werden die ausgefallenen braunen Kristallnadeln mit Pentan gewaschen und dann im Hochvakuum getrocknet. Man erhält 66 mg der Titelverbindung, deren elektrische Leitfähigkeit (Pressling) 1,17 S/cm beträgt; Smp. 192 °C. Elementaranalyse gefunden (berechnet) für C₆₀H₃₀N₁₆S₄Fe: C 62,38 (62,17); H 2,76 (2,61); N 18,05 (19,33); Fe 4,87 (4,82); S 10,95 (11,07).

### Beispiel B3: Herstellung eines CT-Komplexes aus Tetrathiofulvalen und 6,13-Dithio-5,7,12,14-tetracyanoiminpentacen

Zu einer 150 °C heissen Lösung von 100 mg (0,212 mMol) 6,13-Dithio-5,7,12,14-tetracyanoiminpentacen in 50 ml Anisol wird eine gleich warme Lösung von 43 mg (0,212 mMol) Tetrathiofulvalen in 5 ml Anisol gegeben. Man lässt die resultierende Lösung abkühlen und der CT-Komplexe wird abfiltriert. Danach werden die ausgefallenen schwarzen Kristallnadeln mit Pentan gewaschen und dann im Hochvakuum getrocknet. Man erhält 93 mg der Titelverbindung, deren elektrische Leitfähigkeit (Pressling) 0,20 S/cm beträgt; Smp. 229 °C. Elementaranalyse gefunden (berechnet) für C₃₀H₁₂N₈S₆ + 0,5 Anisol: C 53,61 (55,05); H 2,16 (2,21); N 14,97 (15,33); S 27,21 (26,32).

### Beispiel B4: Herstellung eines CT-Komplexes aus Tetramethyltetrathiofulvalen und 6,13-Dithio-5,7,12,14-tetracyanoiminpentacen

Zu einer 150 °C heissen Lösung von 100 mg (0,212 mMol) 6,13-Dithio-5,7,12,14-tetra-cyanoiminpentacen in 50 ml Anisol wird eine gleich warme Lösung von 55 mg (0,212 mMol) Tetramethyltetrathiofulvalen in 5 ml Anisol gegeben. Man lässt die resultierende Lösung abkühlen und der CT-Komplexe wird abfiltriert. Danach werden die ausgefallenen schwarzen Kristallnadeln mit Pentan gewaschen und dann im Hochvakuum getrocknet. Man erhält 87 mg der Titelverbindung, deren elektrische Leitfähigkeit (Pressling) 1,04 S/cm beträgt; Smp. 198 °C. Elementaranalyse gefunden (berechnet) für C₃₄H₂₀N₈S₆ + 0,5 Anisol: C 58,10 (58,65); H 2,77 (2,56); N 17,21 (17,79); S 20,81 (20,36).

## Patentansprüche

1. Verbindungen der Formel I 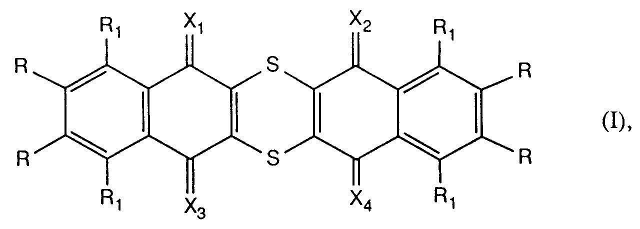 worin die R gleich oder verschieden sind und für H oder C₁-C₄-Alkyl stehen, oder die benachbarten R zusammen -(CH₂)₃- oder -(CH₂)₄- darstellen; die R₁ gleich oder verschieden sind und H oder C₁-C₄-Alkyl bedeuten; und X₁ =N-CN darstellt und X₂, X₃ und X₄ =O oder =N-CN bedeuten.

2. Verbindungen der Formel I gemäss Anspruch 1, dadurch gekennzeichnet, dass R C₁-C₄-Alkyl bedeutet und R₁ für H steht.

3. Verbindungen der Formel I gemäss Anspruch 1, dadurch gekennzeichnet, dass R₁ C₁-C₄-Alkyl bedeutet und R für H steht.

4. Verbindungen der Formel I gemäss Anspruch 1, dadurch gekennzeichnet, dass R und R₁ in der Bedeutung von Alkyl für Methyl oder Ethyl stehen.

5. Verbindungen der Formel I gemäss Anspruch 1, dadurch gekennzeichnet, dass R und R₁ I für H, Methyl oder Ethyl stehen.

6. Verbindungen der Formel I gemäss Anspruch 1, dadurch gekennzeichnet, dass R und R₁ I für H stehen.

7. Verbindungen der Formel I gemäss Anspruch 1, dadurch gekennzeichnet, dass X₁ und X₄ =N-CN und X₂ und X₃ =0 oder =N-CN, oder X₂ und X₃ =N-CN und X₁ und X₄ =O oder =N-CN darstellen.

8. Verbindungen der Formel I gemäss Anspruch 1, dadurch gekennzeichnet, dass X₁, X₂, X₃ und X₄ =N-CN darstellen.

9. Eine Verbindung der Formel I gemäss Anspruch 1, dadurch gekennzeichnet, dass es sich um Dinaphtho[2,3-b;2',3'-e][1,4]dithiin-5,7,12,14-tetracyanoimin handelt.

10. Eine Verbindung der Formel I gemäss Anspruch 1, dadurch gekennzeichnet, dass es sich um 2,3,9,10-Tetramethyl-dinaphtho[2,3-b;2',3'-e][1,4]dithiin-5,7,12,14-tetracyanoimin handelt.

11. Eine Verbindung der Formel I gemäss Anspruch 1, dadurch gekennzeichnet, dass es sich um 2,9-Dimethyl-dinaphtho[2,3-b;2',3'-e][1,4]dithiin-5,7,12,14-tetracyanoimin handelt.

12. Verfahren zur Herstellung von Verbindungen der Formel I gemäss Anspruch 1, dadurch gekennzeichnet, dass man ein Tetraon der Formel Ia 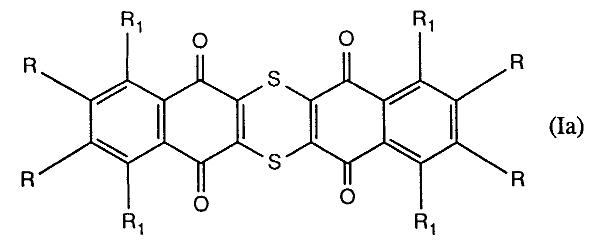 mit äquimolaren Mengen TiCl₄ und [(CH₃)₃SiN]₂C in einer inerten Atmosphäre und in einem inerten Lösungsmittel für 10 bis 30 Stunden erhitzt.

13. Verfahren gemäss Anspruch 12, worin Argon als inerte Atmosphäre verwendet wird.

14. Charge-Transfer Komplexe der Formel II
[(A)ₚ]^{q⊖}B^{q⊕} (II),
worin
a) A das Radikalanion einer Verbindung der Formel I gemäss Anspruch 1 oder einer Mischung von Verbindungen der Formel I ist, und
b) p und q gleich 1 sind und
B das einwertige Radikalkation einer Verbindung der Formel IIIa oder IIIb bedeutet 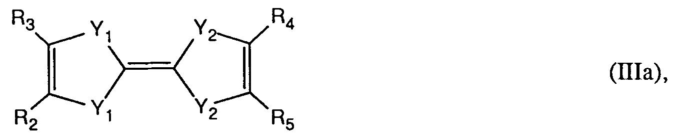 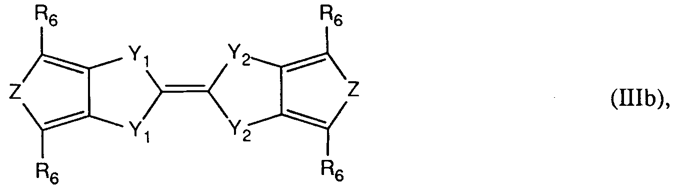 worin R₂, R₃, R₄ und R₅ unabhängig voneinander H, lineares oder verzweigtes C₁-C₁₈-Alkyl-(Z₁)ₙ-, unsubstituiertes oder mit C₁-C₄-Alkyl, C₁-C₄-Alkoxy, C₁-C₄-Alkylthio substituiertes Phenyl-(Z₁)ₙ- ₒder Benzyl-(Z₁)ₙ- bedeuten, oder R₂ und R₃ sowie R₄ und R₅ je zusammen unabhängig voneinander unsubstituiertes oder mit C₁-C₄-Alkyl, C₁-C₄-Alkoxy, C₁-C₄-Alkylthio substituiertes Trimethylen, Tetramethylen, -Z₂-(CH₂)-Z₂-, -Z₂-(CH₂)₂-Z₂-, -Z₁-CH=CH-Z₁- oder -CH=CH-CH=CH- darstellen, n für 0 oder 1 steht, Y₁ und Y₂ unabhängig voneinander -S- oder -Se- sind, Z₁ für -S- oder -Se- steht, Z₂ -O-, -S- oder -Se- darstellt, Z -S-, -Se- oder -NR₇- bedeutet und R₇ H, C₁-C₆-Alkyl, Phenyl oder Benzyl ist, und R₆ für H, C₁-C₄-Alkyl, Phenyl oder Benzyl steht;
oder
das einwertige Radikalkation einer N-aromatischen Verbindung mit insgesamt 1 bis 5 unsubstituierten oder mit Halogen, C₁-C₄-Alkyl oder C₁-C₄-Alkoxy substituierten aromatischen Ringen ist, wobei mindestens ein Ring mindestens eine Gruppe -NR₈- oder [=N⁺R₈-] I⁻ enthält, worin R₈ C₁-C₄-Alkyl oder Benzyl ist;
oder
c) p und q gleich 2 sind und
B das zweiwertige Radikalkation einer wie oben erwähnten N-aromatischen Verbindung ist; oder
d) p gleich 2 und q gleich 1 ist und
B unsubstituiertes Ferrocen oder Fe(Indenyl)₂, oder ein mit C₁-C₆-Alkyl, C₁-C₆-Alkoxy, C₁-C₆-Hydroxyalkyl, -Amino-C₁-C₆-alkyl, Primär- oder Sekundäramino-C₁-C₆-alkyl mit 1 bis 12 C-Atomen in der Primäraminogruppe und 2 bis 12 C-Atomen in der Sekundäraminogruppe, NH₂, Primäramino mit 1 bis 12 C-Atomen, oder Sekundäramino mit 2 bis 12 C-Atomen substituiertes Ferrocen oder Fe(Indenyl)₂ ist.

15. Komplexe gemäss Anspruch 14, dadurch gekennzeichnet, dass R in Formel I C₁-C₄-Alkyl bedeutet und R₁ für H steht.

16. Komplexe gemäss Anspruch 14, dadurch gekennzeichnet, dass R₁ in Formel I C₁-C₄-Alkyl bedeutet und R für H steht.

17. Komplexe gemäss Anspruch 14, dadurch gekennzeichnet, dass R und R₁ in Formel I in der Bedeutung von Alkyl für Methyl oder Ethyl stehen.

18. Komplexe gemäss Anspruch 14, dadurch gekennzeichnet, dass R und R₁ in Formel I für H, Methyl oder Ethyl stehen.

19. Komplexe gemäss Anspruch 14, dadurch gekennzeichnet, dass R und R₁ in Formel I für H stehen.

20. Komplexe gemäss Anspruch 14, dadurch gekennzeichnet, dass X₁ und X₄ =N-CN und X₂ und X₃ =O oder =N-CN, oder X₂ und X₃ =N-CN und X₁ und X₄ =O oder =N-CN darstellen.

21. Komplexe gemäss Anspruch 14, dadurch gekennzeichnet, dass X₁, X₂, X₃ und X₄ =N-CN darstellen.

22. Komplexe gemäss Anspruch 14, dadurch gekennzeichnet, dass in Formel IIIa R₂ und R₃ sowie R₄ und R₅ gleiche Reste darstellen.

23. Komplexe gemäss Anspruch 14, dadurch gekennzeichnet, dass in Formel IIIa R₂ bis R₅ gleiche Reste bedeuten.

24. Komplexe gemäss Anspruch 14, dadurch gekennzeichnet, dass es sich bei R₂ bis R₅ in Formel IIIa in der Bedeutung von Alkyl-(Z₁)ₙ- um C₁-C₁₂-Alkyl-(Z₁)ₙ- handelt, worin Z₁ und n die in Anspruch 14 angegebenen Bedeutungen haben.

25. Komplexe gemäss Anspruch 14, dadurch gekennzeichnet, dass es sich bei dem Rest Alkyl-(Z₁)ₙ- um Methyl, Ethyl, n- und i-Propyl, n-, i- und t-Butyl, Methylthio, Methylseleno, Ethylthio und Ethylseleno handelt.

26. Komplexe gemäss Anspruch 14, dadurch gekennzeichnet, dass Z₁ für -S- und Z₂ für oder -O- oder -S- stehen.

27. Komplexe gemäss Anspruch 14, dadurch gekennzeichnet, dass Y₁ und Y₂ in Formeln IIIa und IIIb -S- oder -Se- bedeuten.

28. Komplexe gemäss Anspruch 14, dadurch gekennzeichnet, dass in den Formeln IIIa und IIIb Y₁ und Y₂ je für -S- stehen.

29. Komplexe gemäss Anspruch 14, dadurch gekennzeichnet, dass R₆ in Formel IIIb H oder C₁-C₄-Alkyl bedeutet.

30. Komplexe gemäss Anspruch 14, dadurch gekennzeichnet, dass Z in Formel IIIb -S- oder -NR₇- bedeutet, wobei R₇ für H oder C₁-C₄-Alkyl steht.

31. Komplexe gemäss Anspruch 14, dadurch gekennzeichnet, dass es sich um solche handelt, worin in Formel I R H, Methyl oder Ethyl bedeutet, R₁ H oder Methyl darstellt und X₁ bis X₄ für =N-CN stehen, und in den Formeln IIIa und IIIb R₂ und R₃ sowie R₄ und R₅ oder R₂ bis R₅ gleich sind und H, lineares oder verzweigtes C₁-C₈-Alkyl-(Z₁)ₙ-, unsubstituiertes oder mit C₁-C₄-Alkyl substituiertes Phenyl-(Z₁)ₙ- oder Benzyl-(Z₁)ₙ- bedeuten, oder R₂ und R₃ sowie R₄ und R₅ je zusammen unabhängig voneinander unsubstituiertes oder mit C₁-C₄-Alkyl substituiertes Trimethylen, Tetramethylen, -Z₂-(CH₂)-Z₂-, -Z₂-(CH₂)₂-Z₂-, -Z₁-CH=CH-Z₁- oder -CH=CH-CH=CH- darstellen, n für 0 oder 1 steht, Y₁ und Y₂ für -S- stehen, Z₁ für -S- steht, Z₂ -O- oder -S- darstellt, Z -S- oder NR₇ bedeutet und R₇ H oder C₁-C₄ Alkyl ist, und R₆ für H oder C₁-C₄ -Alkyl steht.

32. Komplexe gemäss Anspruch 31, dadurch gekennzeichnet, dass es sich um solche handelt, worin in Formel I R und R₁ H bedeuten, X₁ bis X₄ für =N-CN stehen, und in den Formeln IIIa und IIIb R₂ bis R₅ gleich sind und H, oder lineares oder verzweigtes C₁-C₄-Alkyl-(Z₁)ₙ- darstellen, oder R₂ und R₃ sowie R₄ und R₅ je zusammen Trimethylen, Tetramethylen, -Z₂-(CH₂)-Z₂-, -Z₂-(CH₂)₂-Z₂-, -Z₁-CH=CH-Z₁- oder -CH=CH-CH=CH- bedeuten, n für 0 oder 1 steht, Y₁ und Y₂ für -S- stehen, Z₁ für -S- steht, Z₂ -O- oder -S- darstellt, Z -S- oder NR₇ bedeutet und R₇ H oder C₁-C₄-Alkyl ist, und R₆ für H oder C₁-C₄-Alkyl steht.

33. Komplexe gemäss Anspruch 14, worin A in Formel II 6,13-Dithio-5,7,12,14-tetracyanoiminpentacen ist, und B Tetrathiofulvalen, Tetramethyltetrathiofulvalen, Tetraethyltetrathiofulvalen, Dimethyl-diethyl-tetrathiofulvalen, Tetra-n-propyl-tetrathiofulvalen, Tetra-n-butyl-tetrathiofulvalen, Tetra(methylthio)tetrathiofulvalen, Tetra(ethylthio)tetrathiofulvalen, Tetra(n-propylthio)tetrathiofulvalen, Tetra(n-butylthio) tetrathiofulvalen, Dimethyl-dimethylthiotetrathiofulvalen, Diethyl-dimethylthiotetrathiofulvalen, Diethylthio-dimethylthiotetrathiofulvalen und Tetraselenofulvalen bedeutet.

34. Komplexe gemäss Anspruch 14, dadurch gekennzeichnet, dass die N-aromatische Verbindung insgesamt 1 bis 3 Ringe und mindestens einen N-aromatischen Ring enthält.

35. Komplexe gemäss Anspruch 34, dadurch gekennzeichnet, dass es sich bei den aromatischen Ringen um Kohlenwasserstoff-Ringe oder N-heterocyclische Ringe mit einem oder zwei N-Atomen handelt.

36. Komplexe gemäss Anspruch 34, dadurch gekennzeichnet, dass B in Formel II Kationen der Formeln IVa bis IVf entspricht: 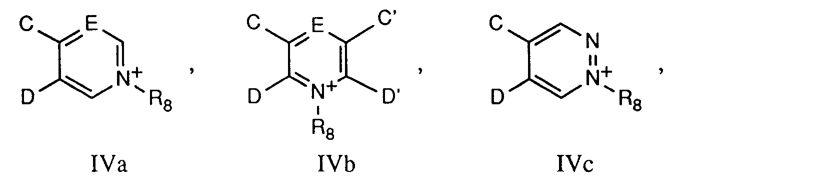 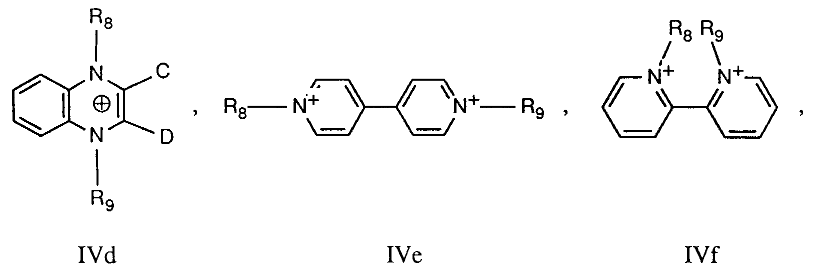 worin C, C', D und D' für H stehen oder C und D und/oder C' und D' die Gruppe -CH=CH-CH=CH- bedeuten, E N oder CH bedeutet, und R₉ unabhängig die gleiche Bedeutung wie R₈ hat, wobei R₈ für C₁-C₄-Alkyl oder Benzyl steht.

37. Komplexe gemäss Anspruch 34, dadurch gekennzeichnet, dass R₈ und R₉ Methyl oder Ethyl bedeuten.

38. Komplexe gemäss Anspruch 34, dadurch gekennzeichnet, dass es sich bei B um Kationen von N-Methyl- und N-Ethyl-pyridinium; N-Methyl- und N-Ethyl-pyrazinium; N-Methyl- und N-Ethyl-chinolinium; N-Methyl- und N-Ethyl-phthalazinium; N-Methyl und N-Ethyl-isochinolinium; N-Methyl- und N-Ethyl-benzopyrazinium; 4,4'-Dimethyl-, 4,4'-Diethyl- und 4-Methyl,4'-ethyl-bipyridinium; N-Methyl- und N-Ethyl-acridinium; N-Methyl- und N-Ethyl-phenazinium; 2,2'-Dimethyl-, 2,2'-Diethyl- und 2-Methyl,2'-ethyl-bipyridinium; N-Methyl- oder N-Ethylpyridazinium; 5,10-Dimethyl-, 5,10-Diethyl-oder 5-Methyl,10-ethyl-5,10-dihydrophenazinium handelt.

39. Komplexe gemäss Anspruch 14, dadurch gekennzeichnet, dass in Formel II A für Dinaphtho[2,3-b;2',3'-e][1,4]dithiin-5,7,12,14-tetracyanoimin steht und B N-Methylpyrazinium, N-Ethylpyrazinium oder 5-Methyl,10-ethyl-5,10-dihydrophenazinium ist.

40. Komplexe gemäss Anspruch 14, dadurch gekennzeichnet, dass es sich bei dem substituierten Ferrocen oder Fe(Indenyl)₂ um eine Verbindung der Formel Fe[R₁₀]₂ handelt, worin R₁₀ für Cyclopentadienyl oder Indenyl steht, die 1 bis 5 beziehungsweise 1 bis 7 Substituenten aus der Gruppe C₁-C₄-Alkyl, C₁-C₄-Alkoxy, C₁-C₄-Hydroxyalkyl, -Amino-C₁-C₄-alkyl, Primär- oder Sekundäramino-C₁-C₄-alkyl mit 1 bis 6 C-Atomen in der Primäraminogruppe und 2 bis 6 C-Atomen in der Sekundäraminogruppe, NH₂, Primäramino mit 1 bis 6 C-Atomen, oder Sekundäramino mit 2 bis 6 C-Atomen enthalten.

41. Komplexe gemäss Anspruch 14, dadurch gekennzeichnet, dass in Formel II A für 6,13-Dithio-5,7,12,14-tetracyanoiminpentacen steht und B unsubstituiertes Ferrocen, Dimethyl-, Tetramethyl-, Hexamethyl-, Octamethyl- oder Decamethylferrocen ist.

42. Verfahren zur Herstellung von CT-Komplexen der Formel II gemäss Anspruch 14, dadurch gekennzeichnet, dass man
a) wenn B Ferrocen, ein Ferrocenderivat oder Fulvalenderivat bedeutet, äquimolare Mengen des Ferrocens beziehungsweise eines Ferrocenderivates oder Fulvalenderivates B und eines 6,13-Dithiopentacenderivates der Formel I in einem inerten organischen Lösungsmittel umsetzt, oder
b) wenn B eine N-aromatische Verbindung bedeutet, 1 Äquivalent eines neutralen 5,10-Dihydrophenazinderivats oder dessen Iodsalz als B oder das Iodsalz einer N-aromatischen Verbindung B mit mindestens 1 Äquivalent eines 6,13-Dithiopentacenderivates der Formel I in einem inerten organischen Lösungsmittel umsetzt.

43. Zusammensetzung, enthaltend a) ein duroplastisches, thermoplastisches oder strukturell vernetztes Polymer und b) einen CT-Komplex der Formel II gemäss Anspruch 14 in Form eines Netzwerks aus Kristallnadeln in der Polymermatrix.

44. Zusammensetzung gemäss Anspruch 43, dadurch gekennzeichnet, dass der CT-Komplex in einer Menge von 0,01 bis 30 Gew.-% enthalten ist, bezogen auf die Zusammensetzung.

45. Zusammensetzung gemäss Anspruch 43, dadurch gekennzeichnet, dass sie als Formkörper, Filme, Folien, Fasern, oder als Beschichtungen auf mindestens einer Oberfläche eines Substrats ausgebildet ist.

46. Verfahren zur Herstellung von Zusammensetzungen gemäss Anspruch 43, dadurch gekennzeichnet, dass man (a) einen CT-Komplex der Formel II einem thermoplastischen Kunststoff einverleibt, (b) einen CT-Komplex der Formel II mindestens einer Komponente eines duroplastischen oder strukturell vernetzbaren Kunststoffs einverleibt und dann die Mischung gegebenenfalls zusammen mit weiteren Komponenten zu einem duroplastischen oder strukturell vernetzten Kunststoff polymerisiert, oder (c) eine Verbindung der Formel I oder Ferrocen oder ein Ferrocenderivat B, ein Fulvalenderivat der Formeln IIIa oder IIIb als B, ein 5,10-Dihydrophenazinderivat oder dessen Iodsalz als B oder das Iodsalz einer N-aromatischen Verbindung B zusammen mit einem thermoplastischen Kunststoff oder mindestens einer Komponente eines duroplastischen oder strukturell vernetzbaren Kunststoffs in einem organischen Lösungsmittel löst, diese Lösung gegebenenfalls zusammen mit weiteren Komponenten für einen duroplastischen oder strukturell vernetzbaren Kunststoff mit einer Lösung Ferrocens oder eines Ferrocenderivats B, eines Fulvalenderivats der Formeln IIIa oder IIIb als B, eines 5,10-Dihydrophenazinderivats oder dessen Iodsalz als B oder eines Iodsalzes einer N-aromatischen Verbindung B beziehungsweise einer Verbindung der Formel I vermischt, das Lösungsmittel entfernt und härtbare Mischungen zu einem duroplastischen oder strukturell vernetzten Kunststoff polymerisiert.

47. Verfahren gemäss Anspruch 46, dadurch gekennzeichnet, dass es mit einer Formgebung verbunden ist.

48. Verwendung der Verbindungen der Formel I gemäss Anspruch 1 als Elektronenakzeptoren.

49. Verwendung der Verbindungen der Formel I gemäss Anspruch 1 zur Herstellung von Charge-Transfer Komplexen.

50. Verwendung der Charge-Transfer Komplexe der Formel II gemäss Anspruch 14 als elektrische Leiter.

51. Verwendung der Zusammensetzung gemäss Anspruch 43 als antistatisch ausgerüstete Formteile für die elektrostatische Abschirmung von Bauteilen oder als antistatisch ausgerüstete Formkörper.

52. Verwendung der Zusammensetzung gemäss Anspruch 43 als elektrische Leiter.

53. Verwendung der Zusammensetzung gemäss Anspruch 43 als Elektrodenmaterial.

54. Verwendung der Zusammensetzung gemäss Anspruch 43 in Form von Filmen oder Folien als Ladungsträger in Kondensatoren.
